(19) Europäisches Patentamt
European Patent Office
Office européen des brevets

(11) Numéro de publication: **0 354 093 B1**

(12) **FASCICULE DE BREVET EUROPEEN**

(45) Date de publication de fascicule du brevet: **22.12.93**

(51) Int. Cl.[5]: **C07D 295/08**, C07D 295/20

(21) Numéro de dépôt: **89402101.3**

(22) Date de dépôt: **25.07.89**

# (54) Dérivés de pipérazine et leur procédé de préparation.

(30) Priorité: **03.08.88 FR 8810481**

(43) Date de publication de la demande:
**07.02.90 Bulletin 90/06**

(45) Mention de la délivrance du brevet:
**22.12.93 Bulletin 93/51**

(84) Etats contractants désignés:
**AT BE CH DE ES FR GB GR IT LI LU NL SE**

(56) Documents cités:
**EP-A- 0 354 094**
**EP-A- 0 364 327**
**US-A- 3 812 127**

(73) Titulaire: **SYNTHELABO**
**22, Avenue Galilée**
**F-92350 Le Plessis Robinson(FR)**

(72) Inventeur: **Manoury, Philippe**
**L'Orée de Verrières,**
**38, Avenue des Vaupépins**
**F-91370 Verrières le Buisson(FR)**
Inventeur: **Binet, Jean**
**12, Hameau de la Gondole**
**F-91650 Breuillet(FR)**
Inventeur: **Obitz, Daniel**
**91, Rue Boucicaut**
**F-92250 Fontenay aux Roses(FR)**
Inventeur: **Defosse, Gérard**
**29, Rue de Tolbiac**
**F-75013 Paris(FR)**
Inventeur: **Dewitte, Elisabeth**
**38, Avenue d'Orgeval**
**F-95210 Saint-Gratien(FR)**
Inventeur: **Veronique, Corinne**
**5, Rue Jacques Duclos, Appt. 4**
**F-94800 Villejuif(FR)**

(74) Mandataire: **Thouret-Lemaitre, Elisabeth et al**
**SYNTHELABO,**
**Service Brevets,**
**B.P. 72**
**F-92352 Le Plessis-Robinson Cédex (FR)**

EP 0 354 093 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente demande a pour objet des dérivés de pipérazine et leur procédé de préparation.

Les composés de la présente invention répondent à la formule générale (I)

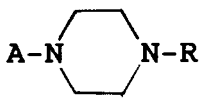

dans laquelle

R est un atome d'hydrogène ou un radical ($C_{1-4}$)alcoxycarbonyle droit ou ramifié,

A est un radical méthoxy-7 naphtalényle-1, un radical méthoxy-6 dihydro-2,3 1H-indényle-1, ou un radical méthoxy-7 tétrahydro-1,2,3,4 naphtalényle-1.

Les sels que forment les composés (I) avec les acides et les énantiomères des composés (I) comportant un carbone asymétrique font partie de l'invention.

Les composés de la présente invention, peuvent être préparés selon le schéma réactionnel donné en annexe :

on fait réagir un composé de formule (II) AX, dans lequel A a la signification donnée ci-dessus et X est un atome d'halogène, avec une [($C_{1-4}$)alcoxycarbonyl]-1 pipérazine (III), dans un solvant tel que MIBC (méthylisobutylcétone), à une température de 20 à 120 °C et, si on souhaite obtenir les composés(I) dans lesquels R est H, on élimine le groupe R alcoxycarbonyle par hydrolyse acide.

Lorsque le composé (I) comporte le radical méthoxy-7 naphtalényle-1 et que le groupe R est un atome d'hydrogène on peut préparer le composé (I) par réaction entre la méthoxy-7 naphtalènamine et la bis-(chloro-2 éthyl)amine sous forme de chlorhydrate.

Les composés de départ (II) sont décrits dans la littérature.

Les composés de l'invention sont des intermédiaires de synthèse, utiles pour préparer des composés à activité thérapeutique, dont certains sont décrits dans le brevet européen EP-0 354 094 correspondant au brevet français 88 10482 (2 635 104) et dans le brevet européen EP-0 364 327 correspondant au brevet français 88 13324 (2 637 591).

Les exemples suivants illustrent l'invention.

Les analyses et les spectres IR et RMN confirment la structure des composés.

Exemple 1.

(Méthoxy-7 naphtalényl-1)-1 pipérazine.

Dans un ballon de 500 ml, surmonté d'un séparateur d'eau de Dean-Stark, placé sous atmosphère d'argon et comportant un agitateur magnétique, on introduit 32 g (0,184 mol) de méthoxy-7 naphtalènamine-1 (Helv. Chim. Acta. 30 816-38, 1947) et 32,84 g (0,184 mol) de chlorhydrate de bis (chloro-2 éthyl)-amine en solution dans 170 ml de butanol. On ajoute une pointe de spatule d'iodure de potassium et chauffe le mélange réactionnel à la température du reflux pendant 20 h.

On ajoute alors 11,6 g (0,092 mol) de carbonate de potassium et laisse refluer 10 h. On ajoute à nouveau 3,87 g (0,03 mol) du même réactif et laisse encore refluer 8 h. On renouvelle deux fois cette opération. On évapore le mélange réactionnel à siccité et triture le résidu entre l'eau et l'éther, on essore un solide mauve. On obtient ainsi 39,2 g (88 %) du chlorhydrate du produit.

On libère la base en agitant le chlorhydrate dans de l'eau, en présence de 20 ml de soude 10N et on extrait le mélange à l'éther. Après séchage et évaporation du solvant , on distille l'huile brute $Eb_{0,1}$ = 200 °C environ.

On obtient finalement 25,5 g (57,2 %) d'une huile incolore dont le spectre RMN confirme la structure.

On prépare le fumarate de cette base et obtient un solide blanc . F 189-191 °C.

Exemple 2.

(±) (Méthoxy-6 dihydro-2,3 1H-indényl-1)-1 pipérazine.

2.1. (±) Méthoxy-6 dihydro-2,3 1H-indénol.

A une solution de 3,8 g (0,1 mol) d'hydrure de lithium et d'aluminium dans 700 ml d'éther sec et 50 ml de tétrahydrofuranne sec, sous atmosphère inerte, on ajoute par portions, en refroidissant 33,5 g (0,2065 mol) de méthoxy-6 dihydro-2,3 1H-indénone. (H.O. House et al. J. Org. Chem. 35 647-51, 1970 et J. Org. Chem. 42,2155-60, 1977).
Après un reflux de 6 h on refroidit dans de la glace et hydrolyse le milieu réactionnel. Après filtration du solide minéral et lavage à l'éther, le filtrat est évaporé à sec. On obtient 33,43 g (98,6 %) d'une huile incolore qui cristallise spontanément en un solide blanc F = 46-47°C.

2.2. (±) Chloro-1 méthoxy-6 dihydro-2,3 1H-indène.

A une solution de 1,64 g (0,01 mol) du composé obtenu précédemment dans 7,5 ml de chlorure de méthylène et 3,5 ml de pyridine, placée dans un bain maintenu à 20°C, on ajoute peu à peu une solution de 2,25 g (0,02 mol) de chlorure de méthanesulfonyle dans 2,5 ml de chlorure de méthylène. Après 2,5 h d'agitation à 20°C, on jette le mélange sur de la glace, décante, lave la phase organique à l'eau acidulée puis à l'eau, la sèche sur sulfate de magnésium, la filtre et la concentre. On obtient 1,63 g (89,5 %) de dérivé chloré.

2.3. (±) (Méthoxy-6 dihydro-2,3 1H-indényl-1)-1 pipérazine.

A une solution de 16 g (0,0859 mol) de pipérazinecarboxylate-1 de diméthyl-1,1 éthyle dans 80 ml de MIBC on ajoute, goutte à goutte, une solution de 8,32 g (0,0455 mol) du dérivé chloré précédent (2.2.) dans 40 ml de MIBC. On ajoute une pointe de spatule d'iodure de sodium et chauffe le mélange réactionnel à la température du reflux pendant 15 h. Après refroidissement on filtre le solide cristallisé, le rince puis évapore le filtrat à siccité. Le résidu est repris entre l'eau et l'éther, et la phase éthérée est lavée puis séchée, filtrée et concentrée. On ajoute au résidu, repris par environ 100ml d'éther, 200 ml d'acide chlorhydrique 3N. On chauffe le mélange à la température du reflux de l'éther. Une fois le dégagement gazeux terminé on décante la phase aqueuse, l'alcalinise par un excès de soude puis on extrait la fraction organique avec de l'éther, lave et sèche l'extrait. Après filtration et concentration on obtient 3,6 g (36 %) d'un solide cristallisé. F = 102-4°C.

Exemple 3.

(±)(méthoxy-7 tétrahydro-1,2,3,4 naphtalényl-1)-1 pipérazine.

3.1. (±) Chloro-1 méthoxy-7 tétrahydro-1,2,3,4 naphtalène.

On agite, à la température ambiante, pendant 3 h 35,6 g (0,2 mole) de (±) méthoxy-7 tétrahydro-1,2,3,4 naphtalénol-1 (D.G. Thomas, A.H. Nathan J. Am. Chem. Soc. 79,331,1948), dans un litre d'acide chlorhydrique concentré..
On extrait le mélange avec de l'héxane, lave la phase organique avec de l'eau, sèche avec du sulfate de magnésium, filtre, et évapore.
On obtient 37,7 g (96 %) d'une huile que l'on utilise brute pour la suite de la réaction.)

3.2 (±) Méthoxy-7 tétrahydro-1,2,3,4 naphtalényl-1)-4 pipérazinecarboxylate de diméthyl-1,1 éthyle.

On chauffe à la température du reflux un mélange de 37,7 g (0,19 mole) du produit précédent avec 29,8 g (0,16 mole) de pipérazinecarboxylate de diméthyl-1,1 éthyle (L.A. Carpino et coll. J. Org. Chem. 48,664,1983) et 55,2 g (0,4 mole) de carbonate de potassium dans 200 ml d'acétone.
On maintient le reflux 72 h. On évapore le mélange, reprend le résidu avec de l'eau et de l'éther, puis décante. On sèche la phase éthérée, filtre, évapore. On purifie le produit par chromatographie sur une colonne de silice. On obtient ainsi 38,9 g d'huile (70 %).

3.3. (±) (Méthoxy-7 tétrahydro-1,2,3,4 naphtalényl-1)-1 pipérazine.

On hydrolyse à 45°C pendant 2 heures 39,6 g (0,11 mole) de (±) (méthoxy-7 tétrahydro-1,2,3,4 naphtalényl-1)-4 pipérazinecarboxylate de diméthyl-1,1 éthyle dans 240 ml d'acide chlorhydrique 3N.
On extrait avec de l'éther, on alcalinise la phase aqueuse, on extrait la base dans de l'éther. On sèche, filtre et évapore la phase éthérée.
On obtient 21 g (75 %) d'huile à partir de laquelle on prépare le fumarate. F = 176°C.

Exemple 4.

(+)(Méthoxy-7 tétrahydro-1,2,3,4 naphtalényl-1)-1 pipérazine.

On solubilise en tiédissant 81 g (0,33 mole) de (±)(méthoxy-7 tétrahydro-1,2,3,4 naphtalényl-1)-1 pipérazine dans 150 ml d'éthanol et 50 g d'acide R(-)mandélique. On laisse la solution refroidir puis filtre le précipité.
On le recristallise deux fois dans de l'éthanol. On obtient le R(-)mandelate de (+)(méthoxy-7 tétrahydro-1,2,3,4 naphtalényl-1)-1 pipérazine F = 174°C $[\alpha]_D^{20}$ = +56,1° c=2 $CH_3OH$. On transforme le sel en base huileuse $[\alpha]_D^{20}$ = +153,0° c=14,25 $CH_3OH$.

Exemple 5.

(-) (Méthoxy-7 tétrahydro-1,2,3,4 naphtalényl-1)-1 pipérazine.

Le filtrat éthanolique précédent (exemple 4) est évaporé, et le résidu est repris avec de l'eau, on alcalinise la phase aqueuse, extrait avec de l'éther. On lave la phase éthérée, sèche, filtre, évapore. On solubilise l'huile obtenue dans 90 ml d'éthanol et 30 g d'acide S(+)mandélique en tiédissant. On laisse le mélange refroidir, filtre le précipité. Après deux recristallisations dans de l'éthanol, on obtient le S(+)-mandélate de (-)(méthoxy-7 tétrahydro-1,2,3,4 naphtalényl-1)-1 pipérazine. F = 174°C $[\alpha]_D^{20}$ =-58,15° c = 2 $CH_3OH$.
$[\alpha]_D^{20}$ =-153,30° c = 14,25 $CH_3OH$ (pour la base).

Les composés (I) de l'invention, préparés à titre d'exemples, sont représentés dans le tableau suivant :

Tableau

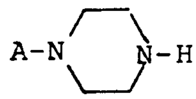

| Composé | A | F(°C)(sel ou base) |
|---|---|---|
| 1 | 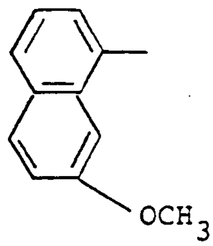 | 189-91 (fumarate) |
| 2 | 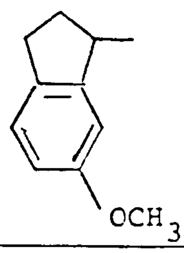 | 102-4 (base) |
| 3 | 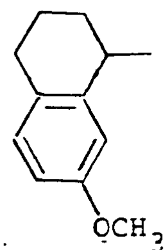 | 176 (fumarate) |
| 4 | 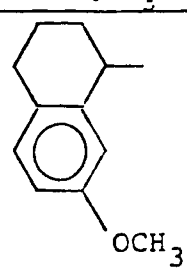 | 174<br>R(-)mandélate de<br>l'isomère dextrogyre |
| 5 | 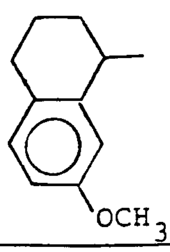 | 174<br>S(+)mandélate de<br>l'isomère lévogyre |

Les composés de l'invention servent d'intermédiaires dans la préparation de divers composés ; en particulier pour la préparation de composés de formule

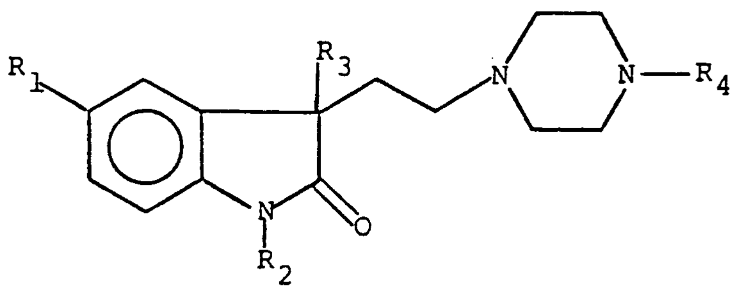

dans laquelle

- $R_1$ est un atome d'hydrogène ou d'halogène ou un radical $(C_{1-4})$alkyle,
- $R_2$ est un atome d'hydrogène ou un radical $(C_{1-4})$alkyle
- $R_3$ est un atome d'hydrogène, un radical $(C_{1-4})$alkyle ou un radical S-$(C_{1-4})$alkyle,
- $R_4$ est un radical méthoxy-7 naphtalènyle-1, méthoxy-6 dihydro-2,3, 1H-indényle-1, ou méthoxy-7 tétrahydro-1,2,3,4 naphtalènyle-1, décrits dans le brevet français 88.10482 et pour la préparation de composés décrits dans le brevet français 88.13324

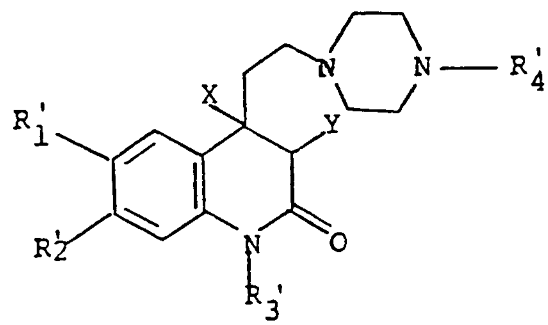

dans laquelle $R'_1$ et $R'_2$ sont chacun, indépendamment l'un de l'autre, un atome d'hydrogène ou d'halogène ou un radical $(C_{1-4})$alkyle,

$R'_3$ est un atome d'hydrogène ou un radical $(C_{1-4})$alkyle,

$R'_4$ est un radical naphtalényle ou tétrahydronaphtalényle, pouvant porter un substituant tel que méthoxy,

X et Y sont chacun un atome d'hydrogène ou forment ensemble une liaison.

Ces composés finals possèdent des propriétés pharmacologiques intéressantes et sont utiles pour le traitement de la migraine, l'anxiété, la dépression, l'obésité, la schizophrénie, les spasmes vasculaires ou gastrointestinaux, l'hypertension et l'agrégation plaquettaire, et comme antiémétiques.

## Annexe

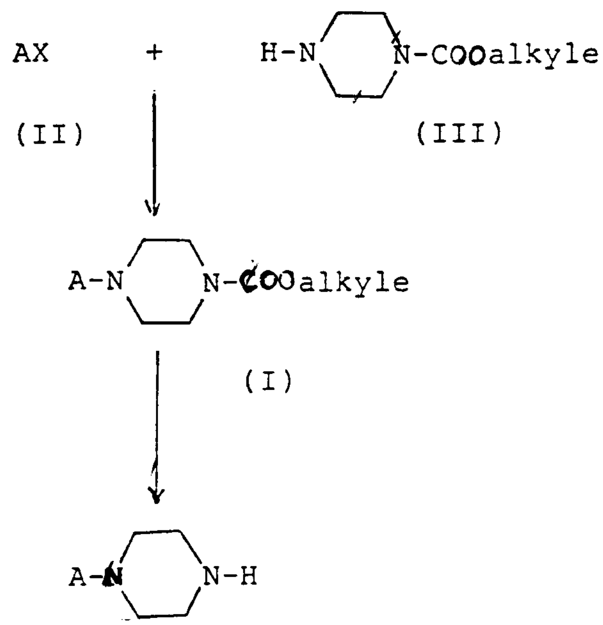

(II) (III)

(I)

(I)

**Revendications**

**Revendications pour les Etats contractants suivants : AT, BE, CH, DE, FR, GB, IT, LI, LU, NL, SE**

**1.** Dérivés de pipérazine répondant à la formule (I)

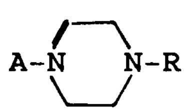 (I)

dans laquelle

R est un atome d'hydrogène ou un radical ($C_{1-4}$)alcoxycarbonyle droit ou ramifié,

A est un radical méthoxy-7 naphtalényle-1, un radical méthoxy-6 dihydro-2,3 1H-indényle-1, ou un radical méthoxy-7 tétrahydro-1,2,3,4 naphtalényle-1,

ainsi que leurs sels d'addition à des acides et leurs énantiomères.

**2.** Procédé de préparation de composés de la revendication 1, procédé caractérisé en ce que l'on fait réagir un composé de formule (II) AX, dans lequel A a la signification donnée ci-dessus et X est un atome d'halogène, avec une [($C_{1-4}$)alcoxycarbonyl]-1pipérazine, et, si on souhaite obtenir les composés (I) dans lesquels R est H, on élimine le groupe R alcoxycarbonyle par hydrolyse acide.

3. Procédé selon la revendication 2, caractérisé par le fait que le solvant utilisé est la méthylisobutylcétone.

4. Procédé selon la revendication 2 ou la revendication 3, caractérisé par le fait que la réaction est effectuée à une température de 20 à 120 ° C.

5. Procédé de préparation du composé (I) selon la revendication 1, dans lequel A est le radical méthoxy-7 naphtalényle-1 et le groupe R est un atome d'hydrogène, procédé caractérisé en ce que l'on fait réagir la méthoxy-7 naphtalènamine et la bis-(chloro-2 éthyl)amine sous forme de chlorhydrate.

6. Procédé de séparation des énantiomères des composés (I) selon la revendication 1, et comportant un carbone asymétrique, procédé caractérisé en ce que l'on fait réagir le composé (I) racémique, avec un acide optiquement actif, tel que l'acide mandélique.

**Revendications pour les Etats contractants suivants : ES, GR**

1. Procédé de préparation de dérivés de pipérazine répondant à la formule (I)

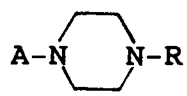

(I)

dans laquelle
R est un atome d'hydrogène ou un radical $(C_{1-4})$alcoxycarbonyle droit ou ramifié,
A est un radical méthoxy-7 naphtalényle-1, un radical méthoxy-6 dihydro-2,3 1H-indényle-1, ou un radical méthoxy-7 tétrahydro-1,2,3,4 naphtalényle-1,
ainsi que de leurs sels d'addition à des acides et de leurs énantiomères, procédé caractérisé en ce que l'on fait réagir un composé de formule (II) AX, dans lequel A a la signification donnée ci-dessus et X est un atome d'halogène, avec une $[(C_{1-4})$alcoxycarbonyl]-1 pipérazine, et, si on souhaite obtenir les composés (I) dans lesquels R est H, on élimine le groupe R alcoxycarbonyle par hydrolyse acide.

2. Procédé selon la revendication 1, caractérisé par le fait que le solvant utilisé est la méthylisobutylcétone.

3. Procédé selon la revendication 1 ou la revendication 2, caractérisé par le fait que la réaction est effectuée à une température de 20 à 120 ° C.

4. Procédé selon la revendication 1, pour la préparation du composé (I) dans lequel A est le radical méthoxy-7 naphtalényle-1 et le groupe R est un atome d'hydrogène, procédé caractérisé en ce que l'on fait réagir la méthoxy-7 naphtalènamine et la bis-(chloro-2 éthyl)amine sous forme de chlorhydrate.

5. Procédé de séparation des énantiomères des composés préparés selon la revendication 1 et comportant un carbone asymétrique, procédé caractérisé en ce que l'on fait réagir le composé (I) racémique avec un acide optiquement actif, tel que l'acide mandélique.

**Claims**
**Claims for the following Contracting States : AT, BE, CH, DE, FR, GB, IT, LI, LU, NL, SE**

1. Piperazine derivatives corresponding to the formula (I)

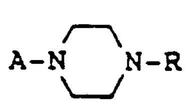

(I)

in which
R is a hydrogen atom or a straight or branched $(C_{1-4})$-alkoxycarbonylradical,
A is a 7-methoxy-1-naphthalenyl radical, a 6-methoxy-2,3-dihydro-1-(1H)-indenyl radical, or a 7-methoxy-1,2,3,4-tetrahydro-1-naphthalenyl radical, as well as their addition salts with acids and their enantiomers.

2. Process for the preparation of compounds of Claim 1, which process is characterized in that a compound of formula (II) AX, in which A has the meaning given above and X is a halogen atom, is reacted with a 1-[$(C_{1-4})$ alkoxycarbonyl]piperazine, and, if it is wished to obtain the compounds (I) in which R is H, the alkoxycarbonyl R group is removed by acid hydrolysis.

3. Process according to Claim 2, characterized in that the solvent used is methyl isobutyl ketone.

4. Process according to Claim 2 or Claim 3, characterized in that the reaction is carried out at a temperature of from 20 to 120 °C.

5. Process for the preparation of the compound (I) according to Claim 1, in which A is the 7-methoxynaphthalenyl radical and the R group is a hydrogen atom, which process is characterized in that 7-methoxynaphthalenamine is reacted with bis-(2-chloroethyl)amine in the form of a hydrochloride.

6. Process for the separation of the enantiomers of the compounds (I) according to Claim 1, and containing an asymmetric carbon atom, which process is characterized in that the racemic compound (I) is reacted with an optically active acid, such as mandelic acid.

**Claims for the following Contracting States : ES, GR**

1. Process for the preparation of piperazine derivatives corresponding to the formula (I)

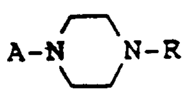

(I)

in which
R is a hydrogen atom or a straight or branched $(C_{1-4})$-alkoxycarbonylradical,
A is a 7-methoxy-1-naphthalenyl radical, a 6-methoxy-2,3-dihydro-1-(1H)-indenyl radical, or a 7-methoxy-1,2,3,4-tetrahydro-1-naphthalenyl radical, as well as their addition salts with acids and their enantiomers, which process is characterized in that a compound of formula (II) AX, in which A has the meaning given above and X is a halogen atom, is reacted with a 1-[$(C_{1-4})$ alkoxycarbonyl]piperazine, and, if it is wished to obtain the compounds (I) in which R is H, the alkoxycarbonyl R group is removed by acid hydrolysis.

2. Process according to Claim 1, characterized in that the solvent used is methyl isobutyl ketone.

3. Process according to Claim 1 or Claim 2, characterized in that the reaction is carried out at a temperature of from 20 to 120 °C.

4. Process according to Claim 1 for the preparation of the compound (I) according to Claim 1, in which A is the 7-methoxynaphthalenyl radical and the R group is a hydrogen atom, which process is characterized in that 7-methoxynaphthalenamine is reacted with bis-(2-chloroethyl)amine in the form of a hydrochloride.

5. Process for the separation of the enantiomers of the compounds prepared according to Claim 1, and containing an asymmetric carbon atom, which process is characterized in that the racemic compound (I) is reacted with an optically active acid, such as mandelic acid.

**Patentansprüche**

**Patentansprüche für folgende Vertragsstaaten : AT, BE, CH, DE, FR, GB, IT, LI, LU, NL, SE**

1. Piperazinderivate der Formel (I)

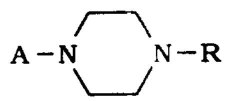

(I)

in der
R ein Wasserstoffatom oder eine geradkettige oder verzweigte $(C_{1-4})$-Alkoxycarbonylgruppe und
A eine 7-Methoxy-naphthalin-1-yl-gruppe, eine 6-Methoxy-2,3-dihydro-1H-inden-1-yl-gruppe oder eine 7-Methoxy-1,2,3,4-tetrahydro-naphthalin-1-yl-gruppe
bedeuten sowie deren Additionssalze mit Säuren und deren Enantiomere.

2. Verfahren zur Herstellung der Verbindungen nach Anspruch 1, **dadurch gekennzeichnet,** daß man eine Verbindung der Formel (II) AX, worin A die oben angegebenen Bedeutungen besitzt und X ein Halogenatom darstellt, mit einem 1-[$(C_{1-4})$-Alkoxycarbonyl]-piperazin umsetzt, und dann, wenn man die Verbindungen (I) herstellen will, worin R H bedeutet, die Alkoxycarbonylgruppe R durch saure Hydrolyse abspaltet.

3. Verfahren nach Anspruch 2, **dadurch gekennzeichnet,** daß das verwendete Lösungsmittel Methylisobutylketon ist.

4. Verfahren nach Anspruch 2 oder Anspruch 3, **dadurch gekennzeichnet,** daß die Reaktion bei einer Temperatur von 20 bis 120 ° C durchgeführt wird.

5. Verfahren nach Anspruch 1 zur Herstellung der Verbindung (I), in der A die 7-Methoxy-naphthalin-1-yl-gruppe und die Gruppe R ein Wasserstoffatom bedeuten, **dadurch gekennzeichnet,** daß man 7-Methoxy-naphthalinamin und Bis(2-chlor-ethyl)-amin in Form des Hydrochlorids umsetzt.

6. Verfahren zur Trennung der Enantiomeren der Verbindungen (I) nach Anspruch 1, die ein asymmetrisches Kohlenstoffatom aufweisen, **dadurch gekennzeichnet,** daß man die racemische Verbindung (I) mit einer optisch aktiven Säure, wie Mandelsäure, umsetzt.

**Patentansprüche für folgende Vertragsstaaten : ES, GR**

1. Verfahren zur Herstellung von Piperazinderivaten der Formel (I)

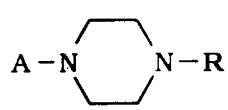

(I)

in der
R ein Wasserstoffatom oder eine geradkettige oder verzweigte $(C_{1-4})$-Alkoxycarbonylgruppe und
A eine 7-Methoxy-naphthalin-1-yl-gruppe, eine 6-Methoxy-2,3-dihydro-1H-inden-1-yl-gruppe oder eine 7-Methoxy-1,2,3,4-tetrahydro-naphthalin-1-yl-gruppe
bedeuten sowie von deren Additionssalzen mit Säuren und deren Enantiomeren, **dadurch gekennzeichnet,** daß man eine Verbindung der Formel (II) AX, worin A die oben angegebenen Bedeutungen besitzt und X ein Halogenatom darstellt, mit einem 1-[$(C_{1-4})$-Alkoxycarbonyl]-piperazin umsetzt, und dann, wenn man die Verbindungen (I) herstellen will, worin R H bedeutet, die Alkoxycarbonylgruppe R durch saure Hydrolyse abspaltet.

2. Verfahren nach Anspruch 1, **dadurch gekennzeichnet,** daß das verwendete Lösungsmittel Methylisobutylketon ist.

3. Verfahren nach Anspruch 1 oder Anspruch 2, **dadurch gekennzeichnet,** daß die Umsetzung bei einer Temperatur von 20 bis 120 ° C durchgeführt wird.

4. Verfahren nach Anspruch 1 zur Herstellung der Verbindung (I), in der A die 7-Methoxy-naphthalin-1-ylgruppe und die Gruppe R ein Wasserstoffatom bedeuten, **dadurch gekennzeichnet,** daß man 7-Methoxy-naphthalinamin und Bis(2-chlor-ethyl)-amin in Form des Hydrochlorids umsetzt.

5. Verfahren zur Trennung der Enantiomeren der Verbindungen (I) nach An spruch 1, die ein asymmetrisches Kohlenstoffatom aufweisen, **dadurch gekennzeichnet,** daß man die racemische Verbindung (I) mit einer optisch aktiven Säure, wie Mandelsäure, umsetzt.